# EUROPEAN PATENT SPECIFICATION

(11) **EP 0 515 085 B1**
(45) Date of publication and mention of the grant of the patent: **27.11.1996**
(21) Application number: 92304317.8
(22) Date of filing: 13.05.1992
(51) Int. Cl.: H04N 5/225

(54) **Cable connecting structure of a solid state image sensor**
Kabel verbindende Struktur für einen Festkörper-Bildsensor
Structure pour la connexion de câble dans un capteur d'images à l'état solide

(30) Priority: 20.05.1991 JP 114767/91
(43) Date of publication of application: 25.11.1992
(73) Proprietor: MATSUSHITA ELECTRIC INDUSTRIAL CO., LTD., Kadoma-shi, Osaka-fu, 571 (JP)
(72) Inventor: Suzuki, Takahisa, Midori-ku, Yokohama-shi (JP); Tsukada, Tatsuki, Hodogaya-ku, Yokohama-shi (JP)
(74) Representative: Sorrell, Terence Gordon

(56) References cited:
- EP-A- 0 080 930
- EP-A- 0 492 349
- FR-A- 2 274 181
- US-A- 4 918 521
- US-A- 4 974 075

## Description

The present invention relates generally to a cable connecting structure of a solid state image sensing apparatus and, more particularly, to a camera cable connecting structure in a separate type camera consisting of a camera head and a camera control unit.

In recent years, there has been an increasing demand for a separate type camera utilising the characteristic that a solid state image sensing device can be easily reduced both in size and in weight. Under such circumstances, there has arisen a new demand from customers for a camera cable connecting method for connecting a camera head to a camera control unit (hereinafter abbreviated to CCU), especially for connecting the camera head with a camera cable. Namely, a direct type of cable fitting is desirable in design terms for the external appearance of a connecting portion between the camera head and the camera cable. For attaching and detaching the camera cable, however, it is desirable to incorporate a cable connector arrangement.

The following is an explanation of a conventional method of connecting the camera head to the camera cable.

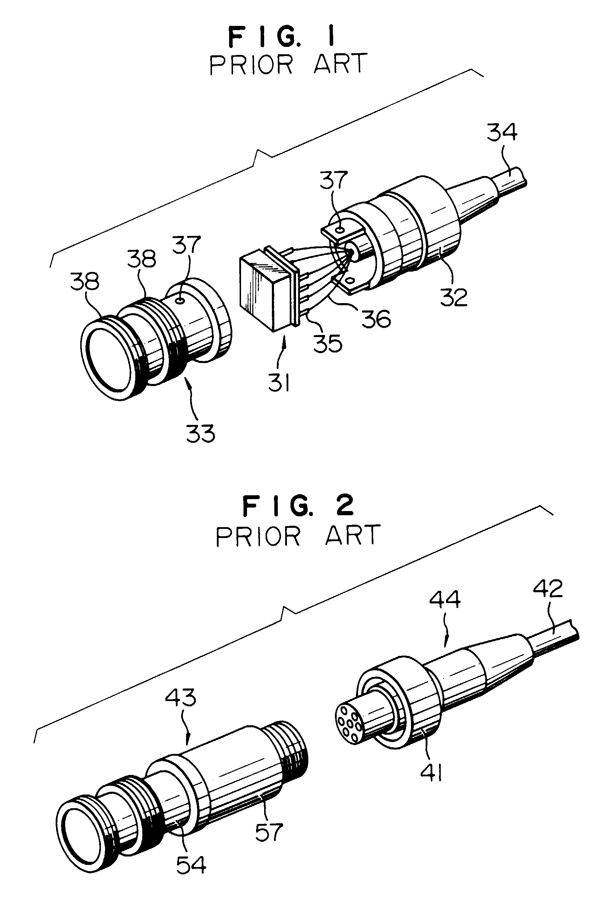

Fig. 1 illustrates a conventional direct type cable fitting connecting method. Designated at 31 in Fig. 1 is an image sensing device module unit having its lead terminals 35 soldered to terminal members 36 of a camera cable 34. Thereafter, the image sensing device module unit is inserted into a body chassis 32 and a sheath chassis 33, and a connecting portion is fastened with a screw 37, whereby they are united. A direct type cable fitting camera head is thus completed. Note that a thread 38 is formed on an outer periphery of the sheath chassis 33; and a lens (not illustrated) is thereby mountable thereto.

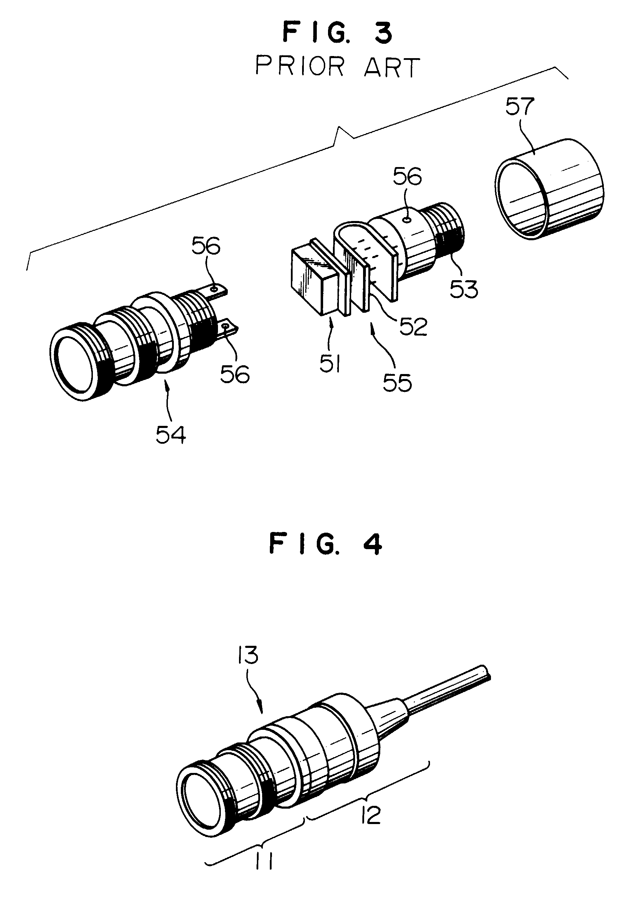

Fig. 2 is another conventional example, showing a connector connecting method. Fig. 3 is a view fully illustrating this method of assembling a camera head. Referring to Figs. 2 and 3, numeral 51 represents an image sensing device module unit to which a connecting plate 52 made of a flexible material is soldered to its lead terminals (not shown). Indicated at 53 is a connector constituting the other end (opposite to a lens mounting side) of the camera head and having at one end the lead terminals soldered to the connecting plate and at its other end a male connector connected to a female connector 41 integrally soldered to the camera cable 42. Numeral 54 denotes a sheath chassis into which a unit 55 consisting of the image sensing device, the connecting plate and the male connector is inserted. These components are fastened with a screw 56, and then the body chassis 57 and the sheath chassis 54 are also fastened with a screw, thus completing the camera head 43. The customer uses the camera by connecting the male connector of the camera head to the female connector 41 of a cable assembly 44.

In the conventional examples described above, however, the external appearance of the camera based on the construction of Fig. 1 satisfies this demand of the customer because of the direct type cable fitting. Attaching and detaching the camera cable, however, involve demounting and mounting by hyperfine soldering. This presents a problem in that this operation is not easy, and the customer's demand can not then be satisfied. Further, in the construction of Fig. 2, the camera cable is attached and detached by the connector system, which facilitates the operation and meets this demand of the customer. In terms of the external appearance of the camera, however, a size of the female connector 41 is substantially equal to that of the camera head 43, which lacks smartness. Thus, a problem arises in that the wishes of the customer can not be satisfied.

Applicant's copending European patent application No. 91121544.0, published as EP-A-0492349, discloses a compact camera head construction. The camera head comprises an image sensing module of identical construction to that of the present invention mounted within a chassis. The module has lead terminals which connect with a female connector located at an end of a cable for transmitting a signal received from an image sensing device chip of the module.

EP-A-0492349, however, comprises a part of the state of the art according to Article 54(3) and (4) EPC since it has a priority date earlier than that of the present application but was published after the filing date of the present application.

It is an object of the present invention to obviate the problems given above by providing a solid sate image sensing apparatus wherein a camera head has a direct type cable fitting from its external appearance, but attaching and detaching of the camera cable are facilitated by a connector structure incorporated into the camera.

To accomplish the object described above, the present invention provides a solid state image sensing apparatus, comprising: a female connector fixed to a cable clamp; a cable connected to cable connecting terminals of said female connector, said cable passing through a through-hole of said cable clamp; an image sensing device module consisting of an image sensing device module unit in which an electrode of an image sensing device chip is connected through a metallic lead to electrodes disposed on side surfaces of device driving circuit boards and lead terminals are disposed on one of said boards, and a sheath chassis encasing said image sensing device module unit; said lead terminals of said image sensing device module transmit a signal from said image sensing device chip to the cable, characterised in that said lead terminals are joined to the female connector and said cable also passes through a through-hole in a body chassis and the body chassis is such that it covers the female connector joined to said lead terminals and the cable clamp.

Therefore, the camera head has a direct type cable fitting structure in terms of its external appearance. When attaching and detaching the cable, the cable can be demounted and mounted through the connector.

Other objects and advantages of the present invention will become apparent from the following description with reference to the drawings, in which:
Fig. 1 is a block diagram illustrating a conventional solid state image sensing apparatus;
Fig. 2 is a sketch drawing of another conventional solid state image sensing apparatus;
Fig. 3 is a view fully depicting a construction of the solid state image sensing apparatus of Fig. 2;
Fig. 4 is a sketch drawing of a solid state image sensing apparatus in accordance with the present invention;
Fig. 5 is a block diagram fully illustrating the solid state image sensing apparatus shown in Fig. 4; and
Fig. 6 is a block diagram fully illustrating an image sensing device module unit shown in Fig. 5.

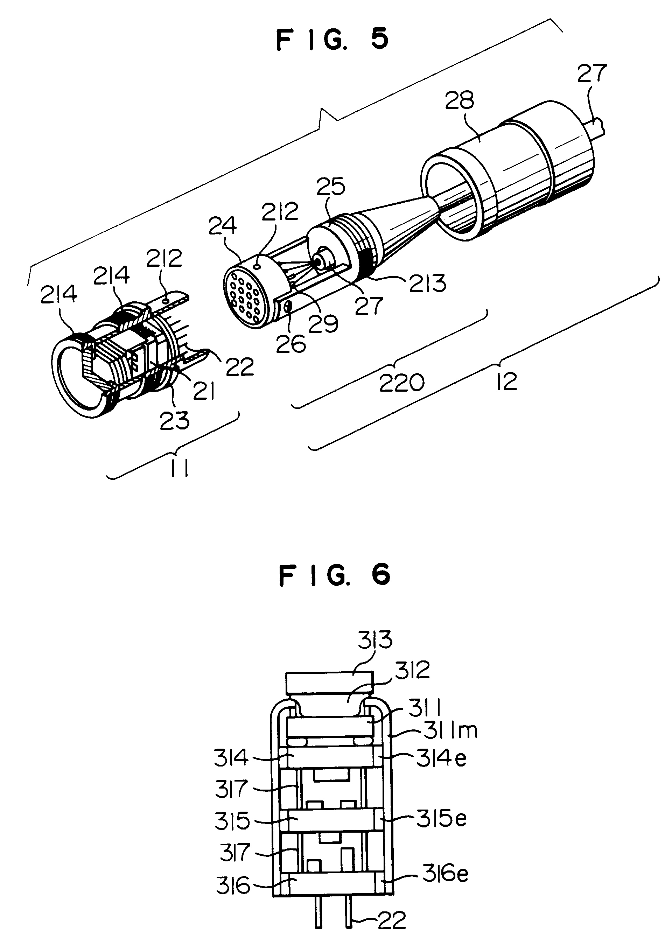

Fig. 4 is a sketch drawing of a solid sate image sensing apparatus in accordance with the present invention. Fig. 5 illustrates a construction of the solid state image sensing apparatus of figure 4. Fig. 6 depicts a construction of an image sensing module unit in the embodiment. Referring to Figs. 4 and 5, numeral 11 designates an image sensing device module; 12 a connector module; 13 a camera head; and 21 an image sensing device module unit consisting of lead terminals 22 comprising a pin function of a male connector, an image sensing device chip, a device driving circuit board and a spatial filter.

Herein, the image sensing device module unit 21 is, as illustrated in Fig. 6, composed of an image sensing device chip 311, an optical glass 313, device driving circuit boards 314,315,316 each packed with a driving circuit for the image sensing device chip 311, and lead terminals 22. The optical glass 313 is fitted to a light receiving surface of the image sensing device chip 311. The device driving circuit boards 314,315,316 are held and fixed with connecting pins 317. Electrodes 314e, 315e,316e are formed on the side portions of the device driving circuit boards 314,315,316. These electrodes are connected to an outer lead 311m connected to a device electrode (unillustrated) of the image sensing device chip 311. Further, metallic lead terminals 22 are attached to the bottom of the device driving circuit board 316.

Turning next to Fig. 5, numeral 23 represents a sheath chassis into which the image sensing device module unit 21 is fixedly inserted. An image sensing device module 11 is thus completed. Designated at 24 is a female connector fixed to a cable clamp 25 with a screw 26. A camera cable generally indicated at 27 passes through through-holes of the body chassis 28 and the cable clamp 25 and is thereafter fixed in a predetermined position in the cable clamp 25; and terminals thereof are soldered to cable connecting terminals 29 of the female connector 24. A connector unit 220 and a connector module 12 are thus completed.

Next, lead terminals pins 22 of the image sensing device module are connector-joined to the female connector 24 of the connector module. After they have been connected by fastening with a screw 212, the body chassis and the cable clamp are screw-fastened and united by engaging a thread (unillustrated) formed inwardly of the body chassis 28 with a thread 213 formed on an outer periphery of the cable clamp, thus completing the camera head 13. Note that a thread 214 is formed on an outer periphery of the sheath chassis 23, whereby a lens (not shown) is mountable thereto.

As discussed above, in accordance with this embodiment, the image sensing device module incorporates the image sensing device module unit in which the image sensing device chip is directly connected to the sheath chassis. The image sensing device module is connected to the connector module in which the body chassis incorporates the connector unit, thus completing the camera head. The camera head therefore has a direct type cable fitting structure in terms of its external appearance. Also provided is the connector structure in which the cable is attachable and detachable simply by demounting the screw fastening portion of the connecting member. Hence, it is possible to provide a camera head structure which sufficiently satisfies the demands of the customer.

Although the illustrative embodiment has been described in detail with reference to the accompanying drawings, it is to be understood that the present invention is not limited to this embodiment. Various changes or modification may be effected therein by one skilled in the art without departing from the scope of the invention as defined by the claims.

## Claims

1. A solid state image sensing apparatus, comprising: a female connector (24) fixed to a cable clamp (25) ; a cable (27) connected to cable connecting terminals (29) of said female connector (24), said cable (27) passing through a through-hole of said cable clamp (25); an image sensing device module (11) consisting of an image sensing device module unit (21) in which an electrode of an image sensing device chip (311) is connected through a metallic lead (311n) to electrodes (314e,315e,316e) disposed on side surfaces of device driving circuit boards (314,315,316) and lead terminals (22) are disposed on one (316) of said boards (314,315,316), and a sheath chassis (23) encasing said image sensing device module unit (21); said lead terminals (22) of said image sensing device module (11) transmit a signal from said image sensing device chip (311) to the cable (27), characterised in that said lead terminals (22) are joined to the female connector (24) and said cable (27) also passes through a through-hole in a body chassis (28) and the body chassis (28) is such that it covers the female connector (24) joined to said lead terminals (22) and the cable clamp (25).

2. A solid state image sensing apparatus as claimed in claim 1, characterised in that the sheath chassis (23) and the female connector (24) are fastened together with a screw (212), said body chassis (28) and said cable clamp (25) are screw-fastened together by engaging a thread formed inwardly of said body chassis with a thread (213) formed on an outer periphery of said cable clamp (25), and thus said body chassis (28) is united with said sheath chassis (23).

## Patentansprüche

1. Festkörper-Bildabtastvorrichtung, aufweisend: eine Steckerbuchse (24), die mit einer Kabelklemme (25) verbunden ist; ein Kabel (27), das mit Kabelverbindungsanschlüssen (29) der Steckbuchse (24) verbunden ist, wobei das Kabel (27) durch ein Durchgangsloch der Kabelklemme (25) hindurchgeht; ein Bildabtastvorrichtungs-Modul (11), das aus einer Bildabtastvorrichtungs-Moduleinheit (21) besteht, in welcher eine Elektrode eines Bildabtastvorrichtungs-Chips (311) durch einen metallischen Leiter (311m) mit Elektroden (314e, 315e, 316e) verbunden ist, die an Seitenflächen von Vorrichtungstreiber-Schaltungskarten (314, 315, 316) angeordnet sind, wobei Leiteranschlüsse (22) auf einer (316) der Karten (314, 315, 316) angeordnet sind; und ein Mantelchassis (23), welches die Bildabtastvorrichtungs-Moduleinheit (21) umhüllt; wobei die Leiteranschlüsse (22) des Bildabtastvorrichtungs-Moduls (11) ein Signal von dem Bildabtastvorrichtungs-Chip (311) zum Kabel (27) übertragen, **dadurch gekennzeichnet,** daß die Leiteranschlüsse (22) mit der Steckbuchse (24) verbunden sind und das Kabel (27) auch durch ein Durchgangsloch in einem Körperchassis (28) hindurchgeht und das Körperchassis (28) derart beschaffen ist, daß es die mit den Leiteranschlüssen (22) und der Kabelklemme (25) verbundene Steckbuchse (24) verdeckt.

2. Festkörper-Bildabtastvorrichtung nach Anspruch 1, dadurch gekennzeichnet, daß das Mantelchassis (23) und die Steckbuchse (24) miteinander durch eine Schraube (212) fest verbunden sind, das Körperchassis (28) und die Kabelklemme (25) miteinander verschraubt sind, und zwar durch in-Eingriff-Bringen eines im Inneren des Körperchassis ausgebildeten Gewindes mit einem am Außenumfang der Kabelklemme (25) ausgebildeten Gewinde (213), und auf diese Weise das Körperchassis (28) mit dem Mantelchassis (23) zusammengefügt ist.

## Revendications

1. Appareil capteur d'images à solide, comprenant: un connecteur femelle (24) fixé à un serre-câble (25); un câble (27) relié à des bornes de connexion de câble (29) dudit connecteur femelle (24), ledit câble (27) passant à travers un trou traversant dudit serre-câble (25); un module formant dispositif capteur d'images (11) constitué d'une unité de module formant dispositif capteur d'images (21), dans laquelle une électrode d'une puce de dispositif capteur d'images (311) est connectée, par l'intermédiaire d'un conducteur métallique (311m), à des électrodes (314e, 315e, 316e) disposées sur des surfaces latérales de plaquettes de circuit de commande (314, 315, 316) du dispositif et des bornes de conducteur (22) sont disposées sur l'une (316) desdites plaquettes (314, 315, 316), et d'un châssis formant gaine (23) qui enveloppe ladite unité de module formant dispositif capteur d'images (21); lesdites bornes de conducteur (22) dudit module formant dispositif capteur d'images (11) transmettant au câble (27) un signal provenant de ladite puce de dispositif capteur d'images (311), caractérisé en ce que lesdites bornes de conducteur (22) sont réunies au connecteur femelle (24) et ledit câble (27) passe également à travers un trou traversant ménagé dans un châssis formant corps (28), et le châssis formant corps (28) est tel qu'il recouvre le connecteur femelle (24) réuni auxdites bornes de conducteur (22) et le serre-câble (25).

2. Appareil capteur d'images à solide tel que défini dans la revendication 1, caractérisé en ce que le châssis formant gaine (23) et le connecteur femelle (24) sont assujettis l'un à l'autre à l'aide d'une vis (212), ledit châssis formant corps (28) et ledit serre-câble (25) sont assujettis l'un à l'autre par vissage, au moyen d'un filetage, formé sur l'intérieur dudit châssis formant corps, qui vient en prise avec un filetage (213) formé sur la périphérie extérieure dudit serre-câble (25), et ledit châssis formant corps (28) est ainsi joint audit châssis formant gaine (23).
